# EUROPÄISCHE PATENTSCHRIFT

(11) **EP 0 415 028 B1**
(45) Veröffentlichungstag und Bekanntmachung des Hinweises auf die Patenterteilung: **13.04.1994**
(21) Anmeldenummer: 90112624.3
(22) Anmeldetag: 03.07.1990
(51) Int. Cl.: C07D 473/18

(54) **Verfahren zur Herstellung von reinem Guanin**
Process for the preparation of pure guanine
Procédé pour la préparation de guanine pure

(30) Priorität: 28.08.1989 DE 3928365
(43) Veröffentlichungstag der Anmeldung: 06.03.1991
(73) Patentinhaber: HÜLS AKTIENGESELLSCHAFT, 45764 Marl (DE)
(72) Erfinder: Steffen, Klaus-Dieter, Dr., D-5202 Hennef 1 (DE)

(56) Entgegenhaltungen:
- EP-A- 0 304 004
- DE-A- 3 235 372
- DE-A- 3 723 874
- DE-A- 3 729 471
- DE-C- 841 751
- PATENT ABSTRACTS OF JAPAN, Band 12, Nr. 365 (C-532)[3212], 29. September 1988
- PATENT ABSTRACTS OF JAPAN, Band 8, Nr. 102 (C-222)[1539], 12. Mai 1984
- BERICHTE DER DEUTSCHEN CHEMISCHEN GESELLSCHAFT, Band 33, 1900, Seiten 1371-1383; W. TRAUBE: "Ueber eine neue Synthese des Guanins und Xanthins"
- CHEMICAL ABSTRACTS, Band 48, Nr. 2, 25. Januar 1954, Columbus, Ohio, USA; R.K. ROBINS et al.: "Purines. II. Synthesis of certain purines and the cyclization of several substituted 4,5-diaminopyrimidines", Seite 1954, Spalte 685
- CHEMICAL ABSTRACTS, Band 109, Nr. 9, 29. August 1988, Columbus, Ohio, USA; M. ENDO et al.: "Process for the purification of guanine as intermediate for drugs", Seite 697, Zusammenfassung-Nr. 73470g
- CHEMICAL ABSTRACTS, Band 100, Nr. 1, 2. Januar 1984, Columbus, Ohio, USA; KOHJIN CO. LTD.: "Purification of guanine", Seite 566, Zusammenfassung-Nr. 6541G

## Beschreibung

Gegenstand der Erfindung ist ein Verfahren zur Herstellung von reinem, farblich verbessertem Guanin aus 2,4,5-Triamino-6-hydroxypyrimidin-Sulfat (TAHP-Sulfat), Natriumformiat und Ameisensäure.

Guanin ist eine Nukleinsäure-Base und wird als Vorprodukt für viele Pharmazeutika eingesetzt. Guanin ist beispielsweise eine Vorstufe für Acyclovir, dem 9-(2-Hydroxyethoxymethyl)-guanin, das gemäß DE-35 44 461 zur Behandlung von Virusinfektionen geeignet ist.

Die Synthese von Guanin aus 2,4,5-Triamino-6-hydroxypyrimidin-Salz durch Kondensation mit einem C₁-Baustein ist lange bekannt. So kann man nach W. Traube, Berichte 33, 1371 (1900) TAHP-Sulfat nach Zusatz einer äquivalenten Menge Natriumformiat in der 8- bis 10fachen Menge 90%iger Ameisensäure 4 bis 5 Stunden kochen. Man erhält dabei Guanin in Ausbeuten von 65 bis 75 % d.Th.

Durch Anwendung von Formamid kann die Reaktionszeit verkürzt und die Ausbeute verbessert werden (DE-A-841751). Nach R.K. Robins et al., J. Am. Chem. Soc. 75, 263 (1953) kann Guanin durch halbstündiges Kochen von TAHPSulfat in Formamid in einer Ausbeute von 96 % erhalten werden. Gemäß DE-3729471 wird Guanin aus TAHP-Sulfat als Suspension in Formamid bei Temperaturen unter 200°C umgesetzt.

Nach JP-A-5916891 wird Guanin aus TAHP-Sulfat und Formaldehyd umgesetzt Allgemein ist es das Ziel, Guanin in hoher Ausbeute und Reinheit herzustellen. Einige Anmeldungen befassen sich nur mit der Entfärbung und Reinigung des rohen Guanins (JP-A-63119484, JP-A-6354373, JP-A-5843973). So wird nach JP 88/54 373 aus TAHP-Sulfat und Formamid hergestelltes Guanin dadurch gereinigt, daß man es in wäßriger Natronlauge löst, mit Aktivkohle behandelt und dann durch eine Säure ausfällt.

Gemäß DE 32 35 372 kann Guanin auch dadurch von Verunreinigungen befreit werden, daß man es als Natriumsalz aus konzentrierter Natronlauge ausfällt.

Nach dem Verfahren von W. Traube mit Ameisensäure ist es nicht möglich, helles, sauberes Produkt in hohen Ausbeuten zu erhalten.

Formamid andererseits hat die Eigenschaft, daß es sich am Siedepunkt von über 200 °C in Kohlenmonoxid und Ammoniak zersetzt. Destilliert man Formamid bei diesen hohen Temperaturen, so werden dabei stark verfärbte Guanin-Rohprodukte erhalten. Formamid erfordert Reaktionstemperaturen von mindestens 150 °C. Auch bei dieser Temperatur ist die Entwicklung von Kohlenmonoxid und Ammoniak beträchtlich. Man erhält außerdem stets Produkte, die einen hohen Reinigungsaufwand erfordern. So müssen zur Herstellung eines farblich ansprechenden Guanins beispielsweise mindestens zwei Reinigungsfällungen und zwei Aktivkohlebehandlungen mit jeweils 50 Gew.-% Aktivkohle, bezogen auf Guanin, durchgeführt werden.

2,4,5-Triamino-6-hydroxypyrimidin ist stark und TAHP-Sulfat ist in geringerem Maße oxidationsempfindlich. Deshalb wird TAHP-Sulfat bei der Hydrierung von 2,4-Diamino-6-hydroxy-5-nitrosopyrimidin gemäß DE 36 38 635 nur in Form violettstichiger Kristalle erhalten. Es ist sehr aufwendig, farbloses TAHP-Sulfat herzustellen. Die Farbe des Guanins, hergestellt nach W. Traube, wird dadurch nicht entscheidend verbessert.

Es bestand daher die Aufgabe, ein nicht zu aufwendiges Verfahren zur Herstellung von Guanin bereitzustellen, bei dem die Kondensation bei Temperaturen von unter 150 °C durchgeführt werden kann und das das Produkt in hoher Ausbeute mit guter Farbe und Reinheit liefert.

Diese Aufgabe wird gemäß Anspruch 1 gelöst. Dabei wird rohes, ungereinigtes, aber wasserfreies TAHP-Sulfat, das mindestens 95%ig ist, und praktisch wasserfreie Ameisensäure mit einem Wassergehalt von weniger als 1 % eingesetzt.

Bei einer Reaktionszeit unter 10 Stunden wird die Ausbeute verschlechtert. Reaktionszeiten über 30 Stunden sind im Prinzip möglich, sie führen jedoch zu keiner weiteren signifikanten Verbesserung. Die Reaktionszeit beträgt bevorzugt 16 bis 25 Stunden.

Die Ameisensäure wird vorzugsweise in der 4- bis 7fachen Gewichtsmenge, bezogen auf das TAHP-Sulfat, eingesetzt. Vorzugsweise verwendet man 95- bis 98%iges TAHP-Sulfat.

Die Reaktion wird in der Regel bei Normaldruck und bei einer Temperatur, die sich einstellt, wenn Ameisensäure unter Rückfluß kocht, durchgeführt. Die Temperatur liegt vorzugsweise bei 105 bis 110 °C. Zur Beschleunigung der Reaktion kann auch ein geringer Überdruck angewendet werden. Der Gesamtdruck liegt deshalb im besonderen bei 1 bis 2 bar.

Als Alkaliformiat wird aus Kostengründen vorzugsweise Natriumformiat verwendet. Das Formiat wird in etwa äquivalenter Menge zum TAHP-Sulfat eingesetzt, also etwa 2 Mole Formiat pro Mol TAHP-Sulfat.

Man kann den Wassergehalt der Reaktionsmischung niedrig halten, wenn man das bei der Kondensationsreaktion entstehende Wasser durch wasserentziehende Mittel wie Acetanhydrid oder Orthoameisensäureester abfängt oder durch azeotrope Schleppmittel entfernt. Man kann das Wasser aber auch nach beendeter Reaktion zusammen mit der Ameisensäure destillieren. Die wasserhaltige Ameisensäure ist sehr rein und kann entweder in anderen Prozessen eingesetzt oder nach Entwässerung wieder zurückgeführt werden.

Der Rückstand der Destillation ist ein Kristallbrei, der im wesentlichen aus Guanin-Formiat, Guanin-Sulfat, Alkalisulfat und Alkaliformiat besteht. Dieser Kristallbrei wird nun in einer wäßrigen Lauge gelöst.

Diese wäßrige alkalische Lösung wird anschließend mit Aktivkohle behandelt. Man setzt dabei 2 bis 30 % Aktivkohle, bezogen auf das Guanin, ein. Die Menge richtet sich vor allem nach dem gewünschten Weißheitsgrad. Bei weniger als 2 % Aktivkohle ist der farbverbessernde Effekt gering. Bei über 30 % Aktivkohle werden die Verluste an Guanin erheblich. Man kann in etwa damit rechnen, daß sich die Ausbeute an Guanin durch eine Behandlung mit 10 % Aktivkohle beispielsweise von 98 % auf etwa 96 % verringert.

Nach Abtrennung der Aktivkohle wird das gelöste Guanin durch Ansäuern mit einer organischen oder anorganischen Säure, vorzugsweise jedoch durch Verseifungsfällung mit Essigsäure- oder Ameisensäureestern entsprechend DE-A-37 23 874, ausgefällt. Auch sauer reagierende Salze oder solche mit einer abpuffernden Wirkung, wie z. B. NH₄Cl, NaHSO₄ oder Natriumacetat, können zur Ausfällung des Guanins eingesetzt werden, denn Guanin fällt bereits bei einem pH-Wert von unter 10 vollständig aus. Nach Filtration, Waschung und Trocknung erhält man das reine, hellfarbige Produkt.

Obwohl bei der Kondensationsreaktion die stöchiometrische Menge Wasser entsteht, ist es erstaunlich und nicht vorhersehbar, daß durch Einsatz wasserfreier Ameisensäure Guanin in sehr guten Ausbeuten von ca. 98 % als Rohprodukt und nach der Reinigung von über 92 % als Endprodukt erhalten wird.
Die Farbqualität des Guanins ist sehr gut, obwohl bei der Reaktion rohres TAHP-Sulfat eingesetzt wird und obwohl bei der Reinigung Aktivkohl in nur begrenzten Mengen verwendet wird.

Das Verfahren erfordert also kein hochreines TAHP-Ausgangsprodukt. Das Verfahren hat darüber hinaus den Vorteil, daß es in einfachen Apparaturen ausgeführt werden kann.

Die Reinheit des Guanins wird entweder durch Titration oder Hochdruckflüssigkeitschromatographie (HPLC) bestimmt und liegt stets über 99,0 %.

Um Farbunterschiede der TAHP-Sulfat- und der Guanin-Qualitäten beurteilen zu können, werden in den folgenden Beispielen Hazen-Farbzahl-Bestimmungen nach DIN 53 409 (APHA-Verfahren) vorgenommen. Zur Farbzahl-Bestimmung wird dabei beim TAHP-Sulfat eine Lösung von 100 mg in 100 ml 1 n HCl und beim Guanin eine Lösung von 5 g in 100 ml 2 n NaOH herangezogen. Die Farbwerte werden entweder durch visuellen Vergleich oder photometrisch mit einem Digitalphotometer bestimmt.

Nachfolgend werden erfindungsgemäße Beispiele mit Zahlen und Vergleichsbeispiele mit Buchstaben gekennzeichnet.

### Beispiel 1

In einem 2-l-Vierhalskolben mit Rührer, Thermometer und Destillationsteil werden 150 g TAHP-Sulfat (0,627 Mol) mit der Farbzahl 5 bis 10 und der Reinheit 97 bis 98 % (nach HPLC) in 750 g Ameisensäure (Wassergehalt: 0,7 %) mit 85,5 g Natriumformiat 20 Stunden unter Rückfluß erhitzt, wobei die Temperatur langsam von 105 auf 110 °C ansteigt. Anschließend werden Ameisensäure und Wasser im Wasserstrahlvakuum vollständig abdestilliert.

Der Rückstand wird in 960 ml Wasser suspendiert und dann mit 180 g 50%iger NaOH gelöst. Die Lösung wird mit 22,5 g Aktivkohle (DEGUSORB^{R}, Fa. Degussa, D-6450 Hanau) versetzt und dann 15 Minuten bei Raumtemperatur gerührt.

Nach Abfiltration der Aktivkohle wird der pH-Wert der Lösung mit 220 g Essigsäureethylester auf unter 10 eingestellt, wobei Guanin ausfällt. Das ausgefallene Guanin wird abfiltriert, mit Wasser gewaschen und dann im Wasserstrahlvakuum bei 40 bis 100 °C getrocknet.
- Ausbeute:: 88,0 g Guanin (92,9 d.Th.)
- Farbzahl:: 109 (photometrisch bei 400 - 500 nm gemessen)
- Reinheit:: 99,8 % (nach HPLC)

### Vergleichsbeispiel A (nach W. Traube, Berichte 33, 1371)

In der in Beispiel 1 beschriebenen Apparatur werden 120 g TAHP-Sulfat (Farbzahl 5 bis 10, Reinheit 97 bis 98 % nach HPLC) mit 68 g Natriumformiat in 1 080 g Ameisensäure (Wassergehalt: 10 %) 4,5 Stunden unter Rückfluß gekocht.

Die Reaktionsmischung wird zur Trockne eingeengt. Der Rückstand wird in 400 ml Wasser aufgenommen, mit 95 ml 50%iger NaOH gelöst und dann mit 18 g Aktivkohle wie in Beispiel 1 behandelt. Nach Abfitration der Aktivkohle wird das Guanin mit Essigsäureethylester ausgefällt, filtriert, mit Wasser gewaschen und getrocknet.
- Ausbeute:: 58,0 g Guanin (76,5 % d.Th.)

Das Produkt ist so stark gelb gefärbt, daß eine APHA-Farbzahl-Bestimmung nicht möglich ist.

### Beispiel 2

Es wird wie in Beispiel 1 verfahren, die Reaktionszeit wird jedoch auf 30 Stunden verlängert. Außerdem wird Guanin aus der wäßrigen Alkalilösung durch eine 50%ige wäßrige NH₄Cl-Lösung ausgefällt.
- Ausbeute:: 87,7 g Guanin (92,5 % d.Th.)
- Farbzahl:: 108 (APHA)

### Vergleichsbeispiel B

Es wird wie in Beispiel 1 verfahren, die Reaktionszeit wird jedoch auf 8 Stunden verkürzt.
- Ausbeute:: 57,3 g Guanin (60,5 % d.Th.)
- Farbzahl:: 506 (APHA)

### Beispiel 3

Der Ansatz von Beispiel 1 wird verdoppelt. Nach einer Reaktionszeit von 20 Stunden und Destillation von Ameisensäure und Wasser wird der Ansatz in 2 gleiche Teile geteilt. In beiden Teilen wird dann wie in Beispiel 1 verfahren, es werden jedoch andere Aktivkohlemengen eingesetzt.

**Tabelle 1**

| Aufarbeitung | % Aktivole, bez. auf Guanin | Farbzahl (APHA) |
|---|---|---|
| a | 15 | 112 |
| b | 20 | 76 |

Gesamtausbeute der beiden Aufarbeitungen:
182,9 g Guanin (96,5 % d.Th.)

### Beispiele 4 und 5, Vergleichsbeispiele C und D

Nach dem Verfahren von Beispiel 1 werden TAHP-Sulfat unterschiedlicher Reinheit und Ameisensäure mit verschiedenen Wassergehalten eingesetzt.

**Tabelle 2**

| Bsp. | TAHP-Sulfat | | % H₂O der Ameisensäure | Guanin | |
|---|---|---|---|---|---|
| | Farbzahl (APHA) | Reinheit nach HPLC (%) | | Ausbeute % d. Th. | Farbzahl (APHA) |
| 4 | 5 | 99 | 0,5 | 92,9 | 85 |
| C | 5 | 99 | 5,8 | 91,4 | 205 |
| 5 | 15 | 98 | 0,7 | 92,2 | 130 |
| D | 15 | 98 | 6 | 90,5 | 203 |

Nach dem erfindungsgemäßen Verfahren wird eine bessere Ausbeute und eine erheblich bessere Farbzahl erhalten.

### Beispiele 6 bis 8

Es wird wie in Beispiel 1 verfahren. Menge und Wassergehalt der Ameisensäure werden jedoch verändert. Zur Reinigung werden außerdem 25 % Aktivkohle, bezogen auf Guanin, eingesetzt.

**Tabelle 3**

| Beispiel | Ameisensäure | | Guanin | |
|---|---|---|---|---|
| | Menge g | % H₂O | Ausbeute % d. Th. | Farbzahl (APHA) |
| 6 | 600 | 0,7 | 95,0 | 129 |
| 7 | 700 | 0,7 | 94,0 | 123 |
| 8 | 700¹⁾ | 0,5 | 94,8 | 136 |

| | | | | |
|---|---|---|---|---|
| ¹⁾ aus vorherigen Ansätzen abdestillierte und entwässerte Ameisensäure | | | | |

## Patentansprüche

1. Verfahren zur Herstellung von reinem, farbverbesserten Guanin durch Umsetzung von 2,4,5-Triamino-6-hydroxypyrimidin-Sulfat mit in etwa äquivalenter Menge Alkaliformiat und der 3- bis 10fachen Gewichtsmenge Ameisensäure,
dadurch gekennzeichnet, daß man
- mindestens 95%iges, wasserfreies 2,4,5-Triamino-6-hydroxypyrimidin-Sulfat einsetzt,
- Ameisensäure mit unter 1 % Wasser verwendet,
- die Reaktion in 10 bis 30 Stunden bei 100 bis 130 °C und 1 bis 3 bar durchführt,
- danach überschüssige Ameisensäure destilliert,
- das Rohprodukt in wäßriger Lösung mit Aktivkohle reinigt und dann
- nach Fällung das Produkt in bekannter Weise isoliert.

2. Verfahren nach Anspruch 1,
dadurch gekennzeichnet,
daß man die 4- bis 7fache Gewichtsmenge Ameisensäure einsetzt.

3. Verfahren nach Anspruch 1,
dadurch gekennzeichnet,
daß man 95- bis 98%iges 2,4,5-Triamino-6-hydroxypyrimidin-Sulfat einsetzt.

4. Verfahren nach Anspruch 1,
dadurch gekennzeichnet,
daß die Reaktionszeit 16 bis 25 Stunden beträgt.

5. Verfahren nach Anspruch 1,
dadurch gekennzeichnet,
daß man einen Druck von 1 bis 2 bar einstellt.

6. Verfahren nach Anspruch 1,
dadurch gekennzeichnet,
daß man als Alkaliformiat Natriumformiat einsetzt.

## Claims

1. A process for preparing pure, improved-colour guanine by reaction of 2,4,5-triamino-6-hydroxypyrimidine sulphate with an approximately equivalent amount of alkali metal formate and from 3 to 10 times the amount by weight of formic acid,
characterized in that
- at least 95 % pure, anhydrous 2,4,5-triamino-6-hydroxypyrimidine sulphate is used,
- formic acid containing less than 1 % of water is used,
- the reaction is carried out in from 10 to 30 hours at from 100 to 130°C and from 1 to 3 bar,
- excess formic acid is then distilled off,
- the crude product is purified in aqueous solution with activated carbon and then
- after precipitation the product is isolated in a known manner.

2. A process according to claim 1,
characterized in that from 4 to 7 times the amount by weight of formic acid is used.

3. A process according to claim 1,
characterized in that from 95 to 98 % pure 2,4,5-triamino-6-hydroxypyrimidine sulphate is used.

4. A process according to claim 1,
characterized in that the reaction time is from 16 to 25 hours.

5. A process according to claim 1,
characterized in that a pressure of from 1 to 2 bar is applied.

6. A process according to claim 1,
characterized in that sodium formate is used as alkali metal formate.

## Revendications

1. Procédé de préparation à l'état pur de guanine de teinte améliorée, par réaction de sulfate de 2,4,5-triamino-6-hydroxy-pyrimidine sur une quantité sensiblement équivalente d'un formiate alcalin et d'une quantité pondérale triple à décuple d'acide formique,
caractérisé par le fait :
- que l'on utilise un sulfate anhydre à 95 % au moins de 2,4,5-triamino-6-hydroxy-pyrimidine,
- que l'on utilise l'acide formique avec moins d'un pour cent d'eau,
- que l'on effectue la réaction en 10 à 30 heures à une température de 100 à 130°C et sous une pression de 1 à 3 bars,
- que l'on distille ensuite l'acide formique en excès,
- que l'on purifie le produit brut en solution aqueuse avec du charbon actif et ensuite
- que l'on isole le produit de manière connue après précipitation.

2. Procédé selon la revendication 1,
caractérisé par le fait qu'on utilise une quantité pondérale quadruple à septuple d'acide formique.

3. Procédé selon la revendication 1,
caractérisé par le fait que l'on utilise du sulfate de 2,4,5-triamino-6-hydroxy-pyrimidine à 95 - 98 %.

4. Procédé selon la revendication 1,
caractérisé par le fait que le temps réactionnel est de 16 à 25 heures.

5. Procédé selon la revendication 1,
caractérisé par le fait qu'on ajuste une pression de 1 à 2 bars.

6. Procédé selon la revendication 1,
caractérisé par le fait que l'on utilise, en tant que formiate alcalin, le formiate de sodium.
